# EUROPEAN PATENT SPECIFICATION

(11) **EP 3 351 615 B1**
(45) Date of publication and mention of the grant of the patent: **29.07.2020**
(21) Application number: 16846606.8
(22) Date of filing: 16.09.2016
(51) Int. Cl.: C12M 1/22, C12M 3/00

(54) **CELL CULTURE VESSEL**
ZELLKULTURGEFÄSS
RÉCIPIENT DE CULTURE CELLULAIRE

(30) Priority: 17.09.2015 JP 2015183779
(43) Date of publication of application: 25.07.2018
(73) Proprietor: AGC Techno Glass Co., Ltd., Haibara-gun, Shizuoka 421-0302 (JP); AGC Inc., Tokyo 100-8405 (JP)
(72) Inventor: MIWA Tatsuaki, Tokyo 100-8405 (JP); IDIRIS Alimjan, Tokyo 100-8405 (JP); ITOH Tohru, Haibara-gun Shizuoka 421-0302 (JP)
(74) Representative: Müller-Boré & Partner Patentanwälte PartG mbB
(86) International application number: PCT/JP2016/077392
(87) International publication number: WO 2017/047735

(56) References cited:
- WO-A1-2007/049576
- WO-A1-2012/036011
- JP-A- 2011 000 131
- US-A1- 2006 013 031
- US-A1- 2006 281 172
- US-A1- 2011 207 215
- US-A1- 2013 323 839
- 'Spheroid Keisei Baiyo-yo Yoki', [Online] 16 July 2015, XP055370332 Retrieved from the Internet: <URL:https://web.archive.org/web/2015071622 0539/http ://www.atg.ushop.jp/rika/hbin/ez2012.html> [retrieved on 2016-11-24]

## Description

### FIELD

The present invention relates to a cell culture vessel for culturing a substance to be cultured such as a cell to obtain a spheroid (cellular aggregate).

### BACKGROUND

Spheroid culture is well known method of artificially culturing cells of human or animal origin in a culture vessel to form three-dimensionally agglutinate. In the spheroid culture, a cell population forms a steric structure and the cells interact with one another. Thus, the cells are considered to be cultured or maintained in a state closer to the three-dimensional structure in a living organism, and the spheroid culture is known to have characteristics superior to ordinary plane adhesive culture. Actually, the spheroid culture is often used for anticancer drug screening using cancer cells, multiplication and differentiation of multipotential stem cells, and so on.

Besides, a known cell culture vessel includes a recess for housing cells and culture fluid at the bottom, the recess having a plurality of microwells on its bottom for assembling the cells by gravity and a side with inclination so as to increase an opening area as it gets closer to its open end (refer to Patent Reference 1).

A suggested cell culture vessel includes two-stage recessed and projecting patterns on the culture surface to constitute a rectangular recess for culturing cells and a two-stage projection arranged in a lattice shape to surround four sides of the recess (refer to Patent Reference 2)_{.} Structures consisting of a bottom, a plurality of wells, a peripheral wall upwardly from the bottom, a partition defining a plurality of regions are also disclosed in Patent References 3-6.

### RELEVANT REFERENCES

### PATENT REFERENCE

Patent Reference 1: JP-A 2015-029431
Patent Reference 2: WO 2007/049576 A1
Patent Reference 3: US 2006/281172 A1
Patent Reference 4: US 2013/323839 A1
Patent Reference 5: US 2006/013031 A1
Patent Reference 6: US 2011/207215 A1

### SUMMARY

### PROBLEMS TO BE SOLVED BY THE INVENTION

However, if an existing cell culture vessel has many wells for enabling mass culture at once, the number of cells with respect to the amount of the culture medium becomes large. This tends to accelerate deterioration of the culture medium (culture fluid) such as its change in pH (hydrogen ion concentration index). As a result, exchange frequency of the culture medium increases. Further, for producing the large amount of spheroids, a culture vessel is required to have wells large in number and its culture surface large in area (for example, a dish having an area of the culture surface of 100 mm).

However, the culture vessel having a large area of the culture surface may cause greater flowage of the culture medium in the culture vessel, during moving the vessel or during sucking and adding the culture medium for its exchange, than a culture vessel having a small area of the culture surface. This may cause the cells or formed spheroids to jump out of the wells and move into other wells, resulting in a decrease in the efficiency of forming the spheroids and difficulty in acquiring spheroids uniform in size.

Hence, the present invention has been made to solve the above problem, and its object is to provide a cell culture vessel that can decrease movement of cells and spheroids between wells due to flowage of a culture medium (culture fluid) in the culture vessel and can culture a large amount of spheroids made uniform in size.

### MEANS FOR SOLVING THE PROBLEMS

A cell culture vessel of the present invention includes a bottom, a peripheral wall, and a partition. The bottom of the cell culture vessel has a culture surface with a plurality of wells. The peripheral wall extends upwardly from a periphery of the bottom. The partition partitions a region on the culture surface surrounded by the peripheral wall into a plurality of sub-regions. , wherein the culture surface between the wells adjacent to each other and between the peripheral wall adjacent to the well and the well is composed of a continuous curved surface without a flat region.

### EFFECT OF THE INVENTION

The present invention can provide a cell culture vessel that can decrease movement of cells and spheroids between wells due to flowage of a culture medium (culture fluid) and can culture a large amount of spheroids made uniform in size.

### BRIEF DESCRIPTION OF THE DRAWINGS

Fig. 1 is a plan view schematically illustrating a cell culture vessel according to a first embodiment of the present invention.
Fig. 2 is a vertical sectional view schematically illustrating a structure of the cell culture vessel in Fig. 1.
Fig. 3 is an enlarged plan view illustrating a periphery of a partition included in the cell culture vessel in Fig. 1.
Fig. 4 is a plan view schematically illustrating another cell culture vessel having a partition different in structure from the cell culture vessel in Fig. 1.
Fig. 5 is a plan view schematically illustrating another cell culture vessel having a partition different in structure from the cell culture vessels in Fig. 1 and Fig. 4.
Fig. 6 is a plan view schematically illustrating another cell culture vessel having a partition different in structure from the cell culture vessels in Fig. 1, Fig. 4 and Fig. 5.
Fig. 7 is a plan view schematically illustrating another cell culture vessel having a partition different in structure from the cell culture vessels in Fig. 1 and Fig. 4 to Fig. 6.
Fig. 8 is a plan view schematically illustrating another cell culture vessel having a partition different in structure from the cell culture vessels in Fig. 1 and Fig. 4 to Fig. 7.
Fig. 9 is a plan view schematically illustrating a cell culture vessel according to a second embodiment of the present invention.
Fig. 10 is an enlarged plan view illustrating a periphery of a partition included in the cell culture vessel in Fig. 9.
Fig. 11 is a vertical sectional view exemplifying variation of the shape of a slit formed in the partition in Fig. 10.
Fig. 12 is a vertical sectional view exemplifying variation of the shape of an upper end in the partition in Fig. 10.
Fig. 13 is an enlarged plan view illustrating a periphery of another partition different in structure from the partition in Fig. 10.
Fig. 14 is an enlarged plan view illustrating a periphery of another partition different in structure from the partitions in Fig. 10 and Fig. 13.
Fig. 15 is a plan view schematically illustrating a cell culture vessel according to a third embodiment of the present invention.
Fig. 16 illustrates a partition provided in a cell culture vessel in Example 1 of the present invention.
Fig. 17 illustrates a culture test result of Example 1 of the present invention.
Fig. 18 illustrates a culture test result of Comparative Example 1.

### DETAILED DESCRIPTION

Hereinafter, embodiments of the present invention will be described based on the drawings.

### <First Embodiment>

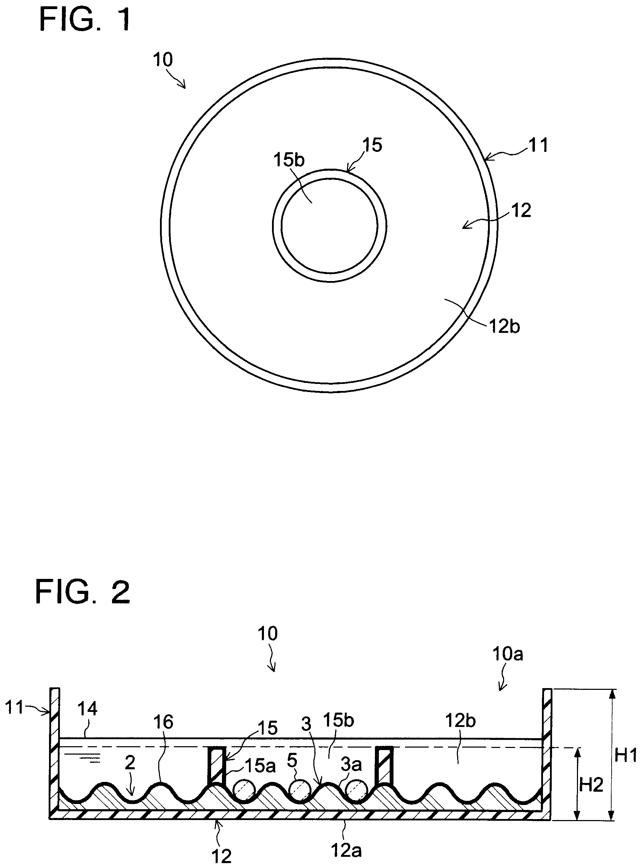

As illustrated in Fig. 1, Fig. 2, a cell culture vessel 10 in this embodiment is a bottomed cylindrical vessel for obtaining spheroids (cellular aggregates) 5 made by three-dimensionally agglutinating cells in a process of culture, while culturing cells being substances to be cultured. As illustrated in Fig. 2, a culture medium (culture fluid) 14 is stored in the cell culture vessel 10. Note that the cell culture vessel 10 can be a vessel of difference form such as a flask or a plate in addition to the cylindrical vessel.

As illustrated in Fig. 1 to Fig. 3, the cell culture vessel 10 mainly includes a bottom 12, a peripheral wall 11, and a partition 15. The bottom 12 is configured in a disk shape and includes a culture surface 3 having a plurality of wells 2. The culture surface 3 is formed on the upper surface of the bottom 12. The culture surface 3 is obtained, for example, by injection molding using a synthetic resin material such as polystyrene.

The peripheral wall 11 extends upwardly from a periphery of the bottom 12. The shape of the peripheral wall 11 is in a state that the periphery is made to stand up. The bottom 12 in a disk shape has a diameter of, for example, 85 mm and a plate thickness of, for example, 1 mm. Further, as illustrated in Fig. 2, the peripheral wall 11 has a height H1 of, for example, 20 mm with reference to the bottom 12 (a mounting surface 12a of the bottom 12 for mounting the body of the cell culture vessel 10). Note that the bottom 12 and the peripheral wall 11 are composed of an integral component. Further, the cell culture vessel 10 may include a lid body for covering an opening partition 10a at the upper end.

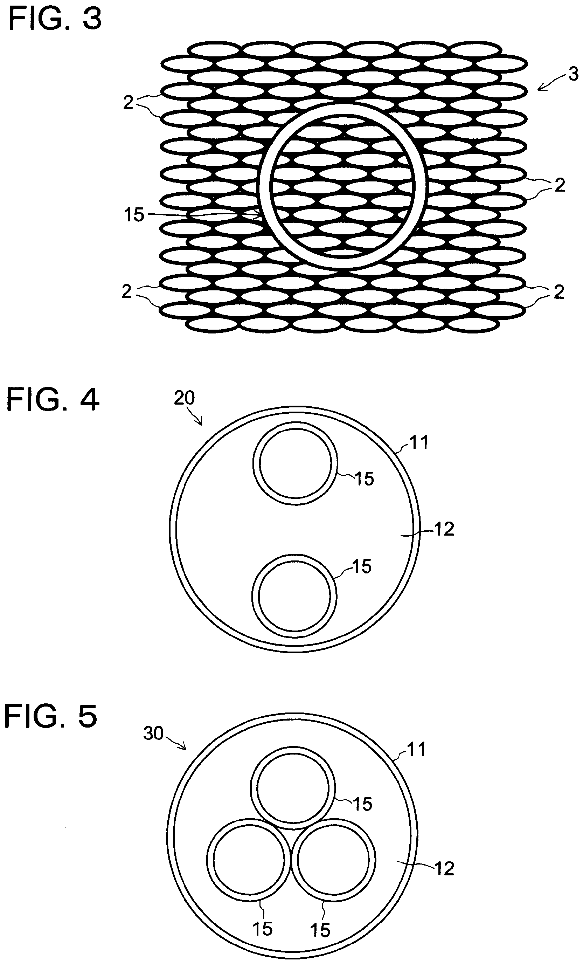

As illustrated in Fig. 2, Fig. 3, the plurality of wells 2 on the culture surface 3 are compartments (recesses) where the spheroids 5 are cultured. The culture surface 3 including the plurality of wells 2 is composed of a continuous curved surface without flat surface. Specifically, since no flat surface exists between the wells (the wells are lined up with no space therebetween), staying of cells between the wells (tops 16) is inhibited (seeded cells surely fall in the wells 2), thereby making it possible to prevent the cells from not becoming spheroids. "The cells not becoming spheroids" here includes monolayer culture, single cell suspension culture, layered culture not forming a spherical shape, the cells being cultured adhering to the culture surface, and the cells not being captured into spheroids but dying in a state of a single cell, and so on.

Further, on the culture surface 3, at least 20 or more wells 2 are formed. More specifically, about 14200 wells 2 (about 250 wells 2/cm²) are formed on the culture surface 3 of the bottom 12 in a disk shape having a diameter of, for example, 85 mm. In one well 2, one spheroid 5 having a desired size is formed.

The wells 2 are formed, for example, by irradiation with laser light toward the culture surface 3 of the bottom 12. The laser irradiation is achieved by applying laser light onto the upper surface (culture surface 3) of the bottom 12.

In more detail, where a plane direction of the bottom 12 is in x-y axes, first, while an irradiation unit of a laser irradiation apparatus is being scanned in a positive direction of the x-axis, the laser light is applied at each regular interval (for example, 800 µm) to form a plurality of wells 2 lined up in the x-axis. Subsequently, the irradiation unit is scanned in the y-axis direction by a fixed distance (for example, 400 µm), and then while the irradiation unit is being scanned in a negative direction of the x-axis, the laser light is applied at each regular interval (for example, 800 µm) to form a plurality of wells 2 lined up in the x-axis. Similarly, the irradiation unit is scanned in the y-axis direction by a fixed distance (for example, 400 µm). The above is repeated to form a plurality of wells 2 regularly arranged on the upper surface of the bottom 12.

Besides, the density of the wells on the culture surface 3 is preferable 10 wells 2/cm² or more, and more preferable 10 wells 2/cm²-10000 wells 2/cm². The density is more preferable 15 wells 2/cm²-5000 wells 2/cm², and furthermore preferable 20 wells 2/cm²-1000 wells 2/cm². Note that the above-described "-" indicating numerical ranges is used to mean including the numerical values described ahead and behind the "-" as the lower limit value and the upper limit value, and the "-" described hereinafter also has the same meaning.

In this embodiment, when the wells are formed by laser, a CO₂ laser is used as a laser light source, and the laser light is applied by pulse irradiation at an output of 10 W and an irradiation speed of 6100 mm/min. The shape of an irradiation spot is a circle and its diameter is about 400 µm. When the spheroid 5 is too small, a desired physiological function is not generated, whereas when the spheroid 5 is too large, its center becomes necrotic. In consideration of the above, the diameter of the irradiation spot is suitably 20 µm-1500 µm.

In the present invention, the wells 2 is preferably uniform in size on the culture surface 3. The wells 2 differing in size are not preferable because the difference in size causes non-uniformity in the sizes of the formed spheroids. To make the sizes of the wells 2 uniform, the irradiation unit of the laser irradiation apparatus is preferably scanned without changing the output and the irradiation speed of the laser when forming the wells 2 on the culture surface 3.

Upon irradiation of the culture surface 3 (the upper surface of the bottom 12) with the laser light, the synthetic resin material constituting the bottom 12 dissolves and vaporizes to form the wells 2 having extremely smooth surfaces. Further, around openings of the wells 2, the dissolved synthetic resin material may heap to form banks. Two wells 2 adjacent to each other and the peripheral wall 11 adjacent to the well 2 are formed via one or a plurality of banks. As illustrated in Fig. 2, no flat surface remains on the tops 16 located between the wells 2 adjacent to each other and between the peripheral wall 11 adjacent to the well 2 and the well 2. In other words, the culture surface between the wells 2 adjacent to each other and between the peripheral wall 11 adjacent to the well 2 and the well 2 is composed of a continuous curved surface without a flat region. Further, the culture surface is made to inhibit cell adhesion and therefore can prevent the cells from not becoming the spheroids as described above.

Besides, adjustment of the irradiation conditions such as the irradiation position and the output amount of the laser light enables adjustment of the distance between the neighboring wells 2, the diameter and depth of the well 2, and the width and height of the bank. In this embodiment, the laser light is applied with the irradiation conditions so as not to leave the flat surface on the culture surfaces 3 between the wells 2 neighboring each other and between the peripheral wall 11 adjacent to the well 2 and the well 2, namely, so as to make the tops 16, between the wells 2 adjacent to each other and between the peripheral wall 11 adjacent to the well 2 and the well 2, a curved surface (non-flat surface) in this embodiment. The laser light is preferably applied onto the entire upper surface of the bottom 12 so that the entire upper surface of the bottom 12 becomes a curved surface (the culture surface 3 having the wells 2). However, a flat surface may be formed at a location on the culture surface 3 that is not used for culture. For example, the periphery of the bottom 12 is a boundary with the peripheral wall 11 and can be difficult to suitably irradiate with the laser light. Accordingly, the periphery of the bottom 12, if located outside the partition 15, may be made a flat surface but not a curved surface (without irradiating the periphery of the bottom 12 with the laser light).

Note that the well 2 has preferably a depth (namely, a depth with reference to the upper surface of the bottom 12 before the irradiation with the laser light) of 10 µm-1500 µm, and has a depth of 200 µm ± 20 µm in this embodiment. The thickness of the bottom 12 is appropriately set according to the depth of the well 2 (so that the recess itself by the well 2 does not penetrate).

The well 2 has preferably a major axis of an opening surface in an elliptical shape of 10 µm-1500 µm, and has a major axis of 500 µm ± 20 µm in this embodiment. Further, the bank (top 16) has preferably a height (height with reference to the upper surface of the bottom 12 before the irradiation with the laser light) of 10 µm-50 µ, and has a height of 25 µm ± 5 µm in this embodiment.

Further, as illustrated in Fig. 2, a coating film (coat layer) 3a for inhibiting adhesion of cells has been formed on the culture surface 3 on the bottom 12 by surface-treating with a cell adhesion inhibitor (protein low adhesive agent). In short, the culture surface 3 is surface-treated to inhibit adhesion of cells. Examples of the cell adhesion inhibitor include phospholipid polymer (2-methacryloyloxyethyl phosphorylcholine or the like), polyhydroxyethyl methacrylate, fluorine-containing compound, polyethyleneglycol and so on. Other than exhibition of the cell adhesion inhibition by the coat layer, a method of molding the culture vessel of a resin having a cell adhesion inhibition effect such as a silicone resin may be employed. The coating film 3a formed on the culture surface 3 including the inner surfaces of the wells 2 prevents the cells from adhering to the culture surface, thereby facilitating agglutination of cells to form spheroids and taking of the spheroids 5 out of the wells 2.

Next, the partition 15 will be described. As illustrated in Fig. 1 to Fig. 3, the partition 15 is formed in an almost cylindrical shape and placed on the bottom 12 (culture surface 3) in the disk shape. The partition 15, which is continuously formed in a substantially cylindrical shape, is composed of a component separable from the bottom 12 and the peripheral wall 11, and formed of a material as same as or different from their materials. On one end (lower end) of the partition 15 in the almost cylindrical shape is joined onto the culture surface 3 of the bottom 12, for example, by a method such as bonding, as illustrated in Fig. 2. Thus, the other end (upper end) of the partition 15 is placed toward the opening 10a at the upper end of the body of the cell culture vessel 10. As described above, the partition 15 is composed of a component separable from the bottom 12 and the peripheral wall 11, thus the number, the shapes, the positions and so on of the partitions 15 on the cell culture vessel can be appropriately adjusted according to the specifications of the cell culture vessel. Besides, after the wells 2 are formed by the above-described laser machining, the partition 15 may be joined to the bottom 12 through the wells 2. In this case, the presence of the wells increases the adhesive strength between the bottom and the partition. Further, in place of the above, the partition 15 is joined to the bottom 12, and then the wells 2 may be formed by the laser machining. In the latter case of the laser machining after the partition 15 is joined, the partition 15 is joined to the flat surface on the bottom 12 before the wells 2 (curved surfaces) are formed, so that bonding therebetween can be easy.

Further, the partition 15 is configured, as illustrated in Fig. 2, so that an inner peripheral surface and an outer peripheral surface of the partition 15 are perpendicular to the mounting surface 12a of the bottom 12 (inner and outer diameters on the one end side and inner and outer diameters on the other end side of the partition 15 are the same). More specifically, the partition 15 extends upwardly from the culture surface 3 along the direction perpendicular to the mounting surface 12a of the bottom 12. The partition 15 is preferably placed in a state of vertically standing up with respect to the mounting surface 12a of the bottom 12 as described above.

Note that the partition can also be slightly inclined. Inclining a part or the whole of the partition makes the cells which are sowed and bump against the partition easily fall into wells. The partition in this case is inclined to a direction perpendicular to the mounting surface 12a of the bottom 12 so that the inner diameter on the other end (upper end) side is larger than the inner diameter on the one end (lower end) side. The inclination angle between the mounting surface 12a of the bottom 12 and the partition is preferably in a range of 95 degrees-110 degrees.

Further, as illustrated in Fig. 1 and Fig. 2, the above-described partition 15 partitions the region on the culture surface 3 surrounded by the peripheral wall 11 into a plurality of sub-regions. In this embodiment, the partition 15 partitions the region on the culture surface 3 into a region 15b on the inner peripheral side of the partition 15 and a region 12b sandwiched between the outer peripheral side of the partition 15 and the inner peripheral side of the peripheral wall 11. Accordingly, the partition 15 can suppress flowage of the culture medium (culture fluid) in the cell culture vessel 10 during replacing the culture medium or transporting the cell culture vessel 10. This makes it possible to prevent cells or spheroids from jumping out of wells 2 and moving to other wells 2, resulting in that spheroids 5 in a uniform size can be obtained.

Further, as illustrated in Fig. 2, a coating film (coat layer) 15a for inhibiting adhesion of cells has been formed on the surface of the partition 15 by the surface treating with the cell adhesion inhibitor (the cell adhesion inhibitor exemplified in the description of the surface treating on the culture surface 3). In short, the surface of the partition 15 is surface-treated to inhibit adhesion of cells. The coating film 15a formed on the surface of the partition 15 inhibits staying of cells on the surface of the partition 15 (stores the cells in the wells 2 along the surface of the partition 15). This can decrease the cells which do not become spheroids in the wells 2.

Besides, as illustrated in Fig. 2, with reference to the bottom 12 (mounting surface 12a of the bottom 12), a height H2 of the partition 15 is 90% or less of the height H1 of the peripheral wall 11. The height H2 of the partition 15 is preferably in a range of 0.5%-90% of the height H1 of the peripheral wall 11 with reference to the bottom 12 (mounting surface 12a of the bottom 12). This configuration can increase the circulation efficiency of the culture medium (culture fluid) between the region 15b inside the partition 15 and the region 12b outside the partition 15 and inside the peripheral wall 11. With an increase in height of the partition, the circulation of the culture medium deteriorates more, whereas with a decrease in height of the partition, the spheroids in the wells 2 become more likely to move to other wells. Therefore, the above-described height H2 of the partition 15 is preferably in a range of 5-80%, more preferably in a range of 10-70%, furthermore preferably in a range of 15-60%, and most preferably in a range of 20-50% of the height H1 of the peripheral wall 11 with reference to the bottom 12.

Further, an area of the culture surface in one sub-region partitioned by the partition 15 (in this embodiment, the region 15b on the inner peripheral side of the partition 15) is 80% or less of the area of the entire culture surface 3 of the bottom 12. More preferably, the area of the culture surface in the one sub-region partitioned by the partition 15 (in this embodiment, the region 15b on the inner peripheral side of the partition 15) is preferably in a range of 5%-80% of the area of the entire culture surface 3 of the bottom 12. With this configuration, the number of cells relative to the amount of the culture medium (culture fluid) in the cell culture vessel 10 can be reduced. This retards deterioration of the culture medium such as variation in pH of the culture medium due to accumulation of waste products resulting from culture or the like in the process of culturing cells, thus the exchange frequency of the culture medium can decrease.

Besides, the partition 15 may be formed using a material through which the culture medium 14 can pass, for example, a membrane (porous film). In this case, the circulation efficiency of the culture medium (culture fluid) 14 between the region 15b and the region 12b partitioned by the partition 15 can increase. Further, the partition 15 may be formed of a material containing a light-blocking coloring agent (for example, titanium oxide exhibiting white, carbon black exhibiting black or the like). More specifically, in the case where the partition 15 is formed of the material containing the light-blocking coloring agent, the visibility at the time of fluorescence observation under a microscope of the cells and the spheroids 5 cultured in the cell culture vessel 10 can be improved. Note that the partition 15, the bottom 12, and the peripheral wall 11 can be formed using the same material such as a glass, as described above.

Here, the method of forming the spheroid 5 using the cell culture vessel 10 according to this embodiment will be described. A cell suspension mixed uniformly is added into the region 15b up to a height not exceeding the partition 15. Then, the cell culture vessel 10 is left to stand to some extent, and when cells fall down to the bottom of the cell culture vessel, the culture medium 14 is added into the region 15b slowly in a manner not to cause the cells to float to above the upper end of the partition 15. Further, as illustrated in Fig. 2, the culture medium 14 is poured into the cell culture vessel 10 so that the liquid level of the culture medium 14 is located above the upper end of the partition 15. Thus, the cells fit in the wells 2 in the region 15b on the inner side of the partition 15. Alternatively, the cell suspension is added into the region 15b to a height not exceeding the partition 15, and the cells are cultured for several hours-several days to form spheroids 5, and then the culture medium 14 may be poured into the cell culture vessel 10 so that the liquid level of the culture medium 14 is located above the upper end of the partition 15.

Thereafter, in this state, the cells are cultured (incubated) for several hours-several days in the cell culture vessel 10 in the cell culture apparatus kept, for example, at 37°C under saturated steam in a 5% carbon dioxide gas atmosphere. Since the coating film 3a using the cell adhesion inhibitor is formed on the inner surface of the well 2, the cells in the well 2 adhere to each other without adhering to the vessel to form a spheroid (cellular aggregate). In this event, the cells three-dimensionally agglutinate according to the shape and size of the well 2 to form the spheroid 5. Thereafter, the culture is further continued, the cells constituting the spheroid 5 multiply and differentiate to exhibit arbitrary bioactive activity.

As has been described, the cell culture vessel 10 in this embodiment can reduce the exchange frequency of the culture medium 14 and culture a large amount of the spheroids 5 made uniform in size. Further, in place of the cell culture vessel 10, a cell culture vessel 20 can be used as an embodiment in which a pair of partitions 15 having the above-described structure are opposite to and away from each other on the bottom 12 (culture surface), as illustrated in Fig. 4.

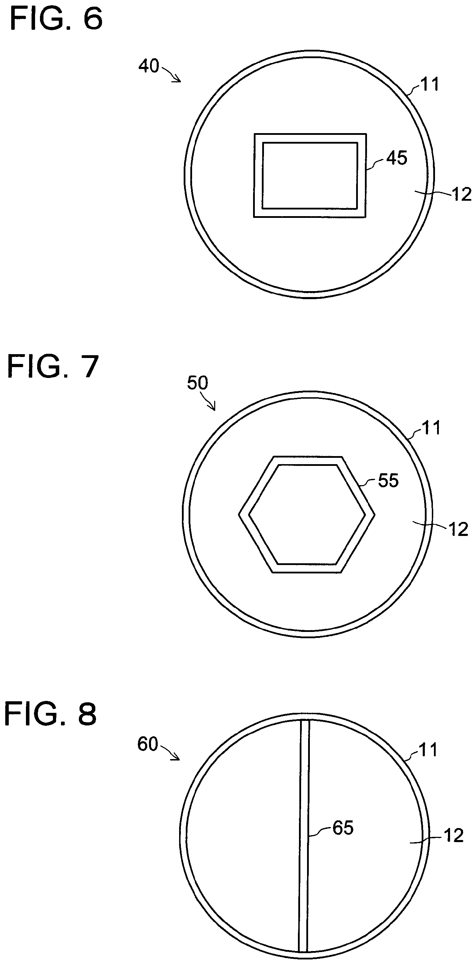

Further, a cell culture vessel 40 of an embodiment includes a partition 45 in a rectangular pipe shape arranged at a center on the bottom 12 as illustrated in Fig. 6, and a cell culture vessel 50 of an embodiment includes a partition 55 in a polygonal pipe shape made by hollowing a polygonal column such as a hexagonal column and arranged at a center on the bottom 12 as illustrated in Fig. 7. Further, a cell culture vessel 60 of an embodiment includes a partition 65 in a flat plate shape connected to two locations on the inner peripheral surface of the peripheral wall 11 on the bottom 12 in a manner to partition the region in a column shape on the bottom 12 (culture surface) surrounded by the peripheral wall 11 into a pair of semicylindrical shaped sub-regions as illustrated in Fig. 8.

### <Second Embodiment>

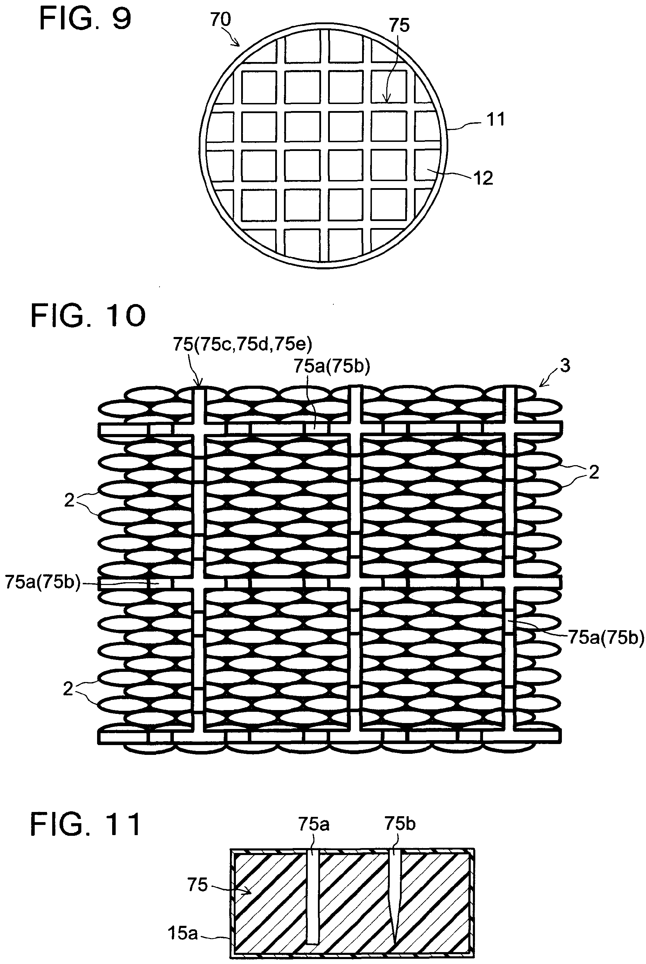

Next, a second embodiment will be described mainly based on Fig. 9 to Fig. 12. Note that in Fig. 9 to Fig. 12, the same components as the components in the first embodiment illustrated in Fig. 1 to Fig. 3 are denoted by the same signs to omit duplicate description.

As illustrated in Fig. 9 to Fig. 12, a cell culture vessel 70 according to the second embodiment includes a partition 75 in place of the partition 15 included in the cell culture vessel 10 in the first embodiment. The partition 75 is formed, as illustrated in Fig. 9, Fig. 10, in a lattice shape as viewing the cell culture vessel 70 from the plane direction. In the partition 75 formed continuously in the lattice shape, a region in a column shape on a bottom 12 (culture surface) surrounded by a peripheral wall 11 is partitioned into a plurality of sub-regions in a lattice shape. Each of ends of the partition 75 is connected to an inner wall surface of the peripheral wall 11.

Further, the partition 75 formed in the lattice shape may have a plurality of slits 75a (75b) as illustrated in Fig. 10, Fig. 11. Fig. 11 exemplifies variation of the shape of the slit. As illustrated in Fig. 11, the slit 75a is formed in a thin-plate shape, whereas the slit 75b is formed in a wedge shape. Both of the slits 75a, 75b may be through type slits opened at both of the upper and lower ends of the partition 75 or may be a non-through type slit opened at the upper end and non-opened at the lower end. To further reduce the flowage of the culture medium near wells, the slits 75a, 75b are preferably the non-through type slits.

The slits 75a, 75b formed in the partition 75 can improve the circulation efficiency of the culture medium (culture fluid) 14 between the regions partitioned in the lattice shape by the partition 75. Note that the cell culture vessel 70 can have a partition in a lattice shape without a slit.

In the case of the partition 75 having the slits, the breadth of the slit is preferably set to 3 mm or less. A breadth of the slit exceeding 3 mm is not preferable because the flowage of the culture medium becomes more likely to occur.

The partition 75 may have one or a plurality of holes instead of the slits. Not-illustrated holes penetrate the partition 75 similarly to the slits 75a, 75b. The positions and the number of the holes are not particularly limited. Further, the diameter of the hole is preferably set to 3 mm or less. A diameter of the hole exceeding 3 mm is not preferable because the flowage of the culture medium becomes more likely to occur. Further, both the hole and slit may be formed.

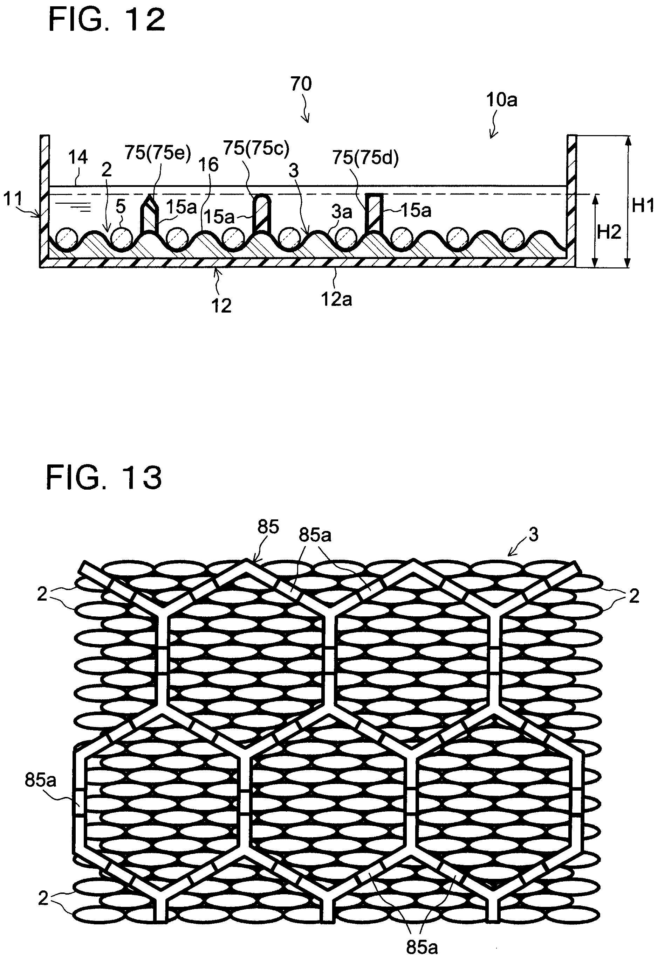

Besides, Fig. 12 exemplifies variation of the shape of an upper end of the partition 75. Examples of the variation of the shape of the partition 75 include a partition 75c having a cross-sectional shape of the upper end formed in a circular shape, a partition 75d having a cross-sectional shape of the upper end formed in a rectangular shape, and a partition 75e having a cross-sectional shape of the upper end formed in a wedge shape as illustrated in Fig. 12.

Further, as illustrated in Fig. 12, the partitions 75 (partitions 75c, 75d, 75e) extend upwardly from the top of a culture surface 3 along a direction perpendicular to a mounting surface 12a of the bottom 12. In other words, the partition 75 is placed in a state of standing up from the top of the culture surface 3 so as to be perpendicular to the mounting surface 12a of the bottom 12.

Here, a method will be described for forming the spheroid 5 using the cell culture vessel 70 according to this embodiment. In the second embodiment, unlike the first embodiment, a cell suspension is added to the cell culture vessel 70 up to a height exceeding the partition 75 and then, without the need to further add the culture medium to the cell culture vessel 70, the cell culture vessel 70 to which the cell suspension has been added can be housed for culture, in the cell culture apparatus set to the conditions as those in the first embodiment.

In the cell culture vessel 70 in this embodiment thus configured, a region on the bottom 12 (culture surface 3) surrounded by the peripheral wall 11 is partitioned into a plurality of comparatively small sub-regions by the partition 75 in the lattice shape, and thus can enhance the effect of suppressing the flowage of the culture medium (culture fluid) in the cell culture vessel 70 in replacing the culture medium and the like. This improves the function of preventing the jumping of cells and spheroids 5 out of the wells 2, resulting in that spheroids 5 more uniform in shape and size can be obtained.

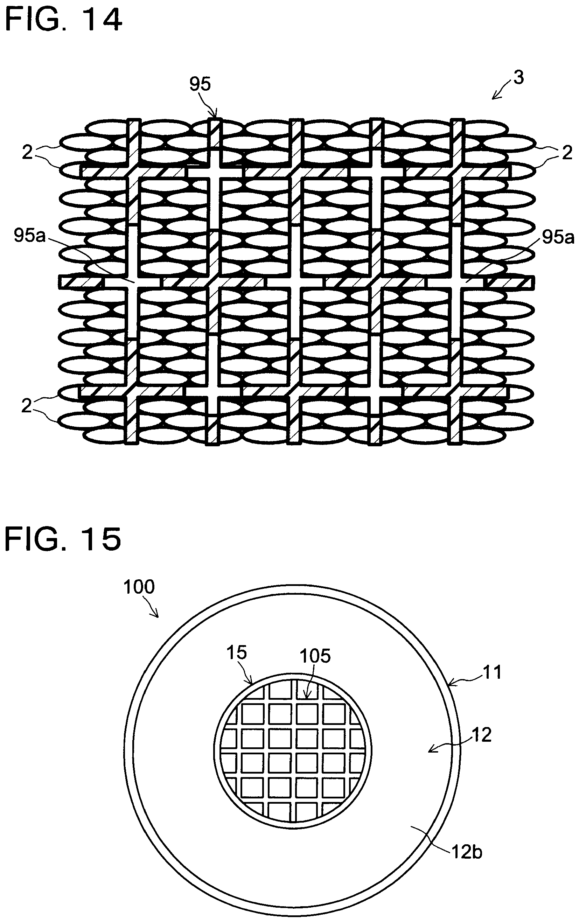

Further, in place of the cell culture vessel 70, a cell culture vessel of an embodiment can include a partition 85 in a honeycomb structure having slits 85a formed on the bottom 12 (culture surface 3) as illustrated in Fig. 13. Further, a cell culture vessel of an embodiment can include a partition 95 in a lattice shape having a partially different structure from that of the partition 75 as illustrated in Fig. 14. In the plan view in Fig. 14, a region without a slit in the partition 95 is hatched for clarifying the difference in structure from the partition 75. More specifically, the partition 95 in the lattice shape is provided with slits 95a at corners where the partition bodies intersect with each other as illustrated in Fig. 14. The cell culture vessel of an embodiment can includes the partition 95.

### <Third Embodiment>

Next, a third embodiment will be described mainly based on Fig. 15. Note that in Fig. 15, the same components as the components in the first and second embodiments illustrated in Fig. 1 to Fig. 14 are denoted by the same signs to omit duplicate description.

As illustrated in Fig. 15, a cell culture vessel 100 according to the third embodiment includes the configuration of the cell culture vessel 10 according to the first embodiment having the partition 15, and a partition 105 in a lattice shape inside the partition 15. The partition 105 has the same structure as that of the partition 75 of the cell culture vessel 70 in the second embodiment illustrated in Fig. 9.

Here, the partition 105 of the cell culture vessel 100 may have the same structure as that of the partition 85 in the honeycomb structure illustrated in Fig. 13 or the same structure as that of the partition 95 illustrated in Fig. 14. Further, the partition 15 of the cell culture vessel 100 can be replaced by the partition 45 illustrated in Fig. 6 or the partition 55 illustrated in Fig. 7. Alternatively, the cell culture vessel 100 may include a plurality of partitions 15 as illustrated in Fig. 4, Fig. 5. In this case, the partition 105 is arranged inside each of the individual partitions 15. Further, the cell culture vessel 100 may include the partition 65 illustrated in Fig. 8. In this case, the partition 105 is arranged in any one of a region on one side of the partition 65 (for example, a right side of the partition 65 in Fig. 8) and a region on the other side of the partition 65 (for example, a left side of the partition 65 in Fig. 8).

The area of the culture surface in one sub-region partitioned by the partition 105 inside the partition 15 provided in the cell culture vessel 100 is 40% or less of the area of the entire culture surface 3 of the bottom 12. More specifically, the area of the culture surface in the one sub-region partitioned by the partition 105 is preferably in a range of 1%-20% of the area of the entire culture surface 3 of the bottom 12. This configuration enables further suppression of the flowage of the culture medium (culture fluid) in the cell culture vessel 100.

Accordingly, the cell culture vessel 100 according to the third embodiment can provide both merits produced by the cell culture vessels according to the first and second embodiments. In other words, the cell culture vessel 100 according to the third embodiment can reduce the exchange frequency of the culture medium and can culture a large amount of spheroids uniform in size, and further can more surely suppress the flowage of the culture medium (culture fluid) in the cell culture vessel in replacing the culture medium.

### Examples

Hereinafter, the present invention will be concretely described using examples, but the present invention is not limited to the following contents.

### (Example 1)

A cell culture vessel manufactured in this example includes, as illustrated in Fig. 13, a partition formed in a honeycomb shape when viewed from the plane direction of the cell culture vessel. This partition partitions the region in the column shape on the bottom (culture surface) surrounded by the peripheral wall into a plurality of honeycomb-shaped sub-regions.

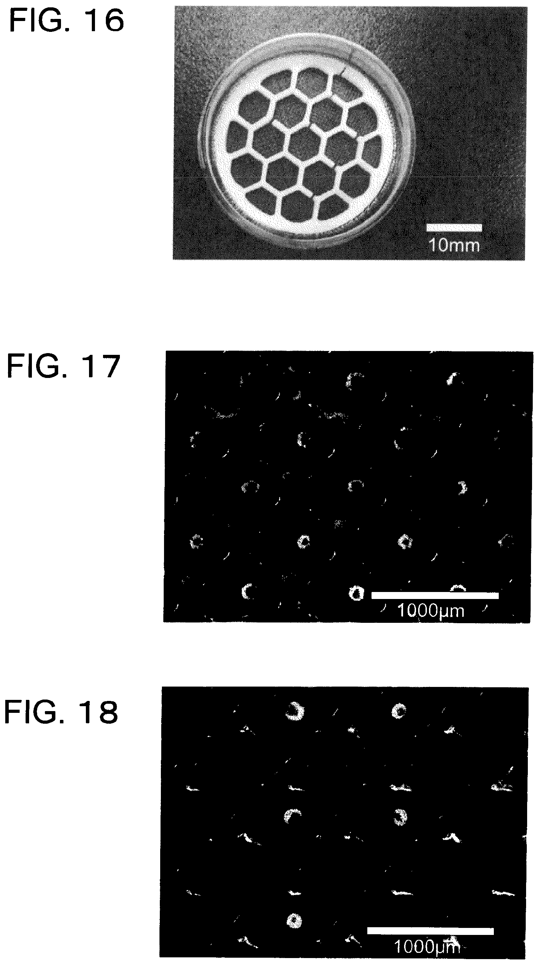

The partition provided in the cell culture vessel was produced by a 3D printer. The structure of the partition was formed in a honeycomb structure to have a width of the honeycomb of 6 mm and a size lying over the entire culture surface of a 35 mm dish. Note that the width of the honeycomb mentioned here means the shortest length between sides facing each other of a honeycomb (hexagon). For the reason of the strength of the partition, a rim of a width of about 4 mm was provided at the outer periphery of the partition. The height of the partition was set to about 1 mm. In the case of the 35 mm dish, the height of the culture medium was generally 2-3 mm, and the partition was designed to completely sink in the culture medium. Fig. 16 illustrates the dish with the produced partition attached thereto. For confirming the effect of the cell culture vessel, a culture test was carried out by the following procedure.

First, the iPS cell 253G1 strain was cultured to about 70% confluent on Matrigel (manufactured by Corning) coat in mTeSR1 (manufactured by STEMCELL TECHNOLOGIES) culture medium. The iPS cell 253G1 strain was treated by Accutase (manufactured by Sigma) at 37°C for 5 minutes, and then an equal amount of mTeSR1 with 10 µM of Y-27632 (manufactured by Wako) added thereto was added to dissociate the iPS cell 253G1 strain into single cells by pipetting. The cells were collected by centrifugal separation, and seeded to a microfabrication culture vessel (manufactured by AGC TECHNO GLASS CO., LTD., EZSPHERE (registered trademark) Type #900 35 mm Dish) with the partition so that the mTeSR1 was 3 mL and the number of cells was 4.8 × 10⁵ per dish to form spheroids.

One day after and two days after start of the culture, the culture vessel was gently taken out of the incubator and moved to a microscope and observed, and then a half amount (1.5 mL) of the culture medium was replaced with new mTeSR1. Subsequently, 3 days after, a half amount of the culture medium was replaced with mTeSR1 with Live/Dead Cell Straining Kit II (manufactured by PromoKine) having a concentration of 2 times added thereto, and incubated for 30 minutes under the condition of 37°C and 5% carbon dioxide gas. Thereafter, fluorescence observation on a bright field and with an excitation wavelength of 470 nm was performed using a fluorescence microscope EVOS FL Auto (manufactured by Life Technologies). The result is illustrated in Fig. 17.

### (Comparative Example 1)

A culture test was carried out by the same method as that in Example 1 except using no partition. Further, fluorescence observation was carried out by the same method as that in Example 1. The result is illustrated in Fig. 18.

The microscope image illustrated Fig. 17 is the example of the present invention, and is the result of culture with a partition having a width of a honeycomb of 6 mm. The microscope image illustrated Fig. 18 is the comparative example of the present invention, and is the result of culture without partition. On the condition of Comparative Example 1, many spheroids jumped out of minute wells. On the other hand, on the condition of Example 1, almost all of the spheroids did not jump out but stayed in wells. This indicates an effect of preventing jumping of spheroids by the partition.

Though the present invention has been described more concretely with embodiments, the present invention is not limited to the embodiments as they are but can be variously changed at the implementation phase without departing from the spirit of the inventions. For example, some components may be omitted from all of the components illustrated in the embodiments, or a plurality of components disclosed in the above embodiments may also be combined as needed.

### EXPLANATION OF REFERENCE NUMERALS

10, 20, 30, 40, 50, 60, 70, 100 ... cell culture vessel, 2 ... well, 3 ... culture surface, 3a, 15a ... coating film, 5 ... spheroid, 10a... opening, 1 1 ... peripheral wall, 12 ... bottom, 12a ... mounting surface, 12b, 15b ... region, 14 ... culture medium, 15, 45, 55, 65, 75, 75c, 75d, 75e, 85, 95, 105 ... partition, 16 ... top, 75a, 75b, 85a, 95a ... slit.

## Claims

1. A cell culture vessel (10) comprising:
a bottom (12) having a culture surface (3) with a plurality of wells; (2);
a peripheral wall (11) extending upwardly from a periphery of the bottom; (12); and
a partition (15) partitioning a region on the culture surface surrounded by the peripheral wall (11) into a plurality of sub-regions,
wherein the culture surface between the wells adjacent to each other and between the peripheral wall adjacent to the well and the well is composed of a continuous curved surface without a flat region.

2. The cell culture vessel according to claim 1, wherein a height of the partition is in a range of 0.5-90% of a height of the peripheral wall with reference to the bottom.

3. The cell culture vessel according to claim 1 or 2, wherein an area of one of the sub-regions is in a range of 5-80% of an area of the region.

4. The cell culture vessel according to any one of claims 1 to 3, wherein the culture surface has wells at a density of at least 10 wells/cm² or more.

5. The cell culture vessel according to any one of claims 1 to 4, wherein the partition has a slit and/or a hole.

6. The cell culture vessel according to any one of claims 1 to 5, wherein the partition is continuously formed.

7. The cell culture vessel according to any one of claims 1 to 6, wherein the partition extends upwardly from the culture surface along a direction perpendicular to a mounting surface of the bottom.

8. The cell culture vessel according to any one of claims 1 to 7, wherein the partition is composed of a component separable from the bottom and the peripheral wall.

9. The cell culture vessel according to any one of claims 1 to 8, wherein the culture surface is surface-treated to inhibit adhesion of cells.

10. The cell culture vessel according to any one of claims 1 to 9, wherein the partition is surface-treated to inhibit adhesion of cells.

11. The cell culture vessel according to any one of claims 1 to 10, wherein the partition is connected to the peripheral wall.

## Patentansprüche

1. Zellkulturgefäß (10), umfassend:
einen Boden (12) mit einer Kulturoberfläche (3) mit einer Vielzahl von Vertiefungen (2),
eine Umfangswand (11), die sich von einem Umfang des Bodens (12) nach oben erstreckt, und
eine Trennwand (15), die einen Bereich auf der Kulturoberfläche, umgeben von der Umfangswand (11), in eine Vielzahl von Unterbereichen unterteilt,
wobei die Kulturoberfläche zwischen den jeweils aneinandergrenzenden Vertiefungen und zwischen der an die Vertiefung angrenzenden Umfangswand und der Vertiefung aus einer kontinuierlich gekrümmten Oberfläche ohne einen flachen Bereich aufgebaut ist.

2. Zellkulturgefäß gemäß Anspruch 1, wobei eine Höhe der Trennwand in einem Bereich von 0,5-90% einer Höhe der Umfangswand bezüglich dem Boden ist.

3. Zellkulturgefäß gemäß Anspruch 1 oder 2, wobei eine Fläche von einem der Unterbereiche in einem Bereich von 5-80% einer Fläche des Bereichs ist.

4. Zellkulturgefäß gemäß einem der Ansprüche 1 bis 3, wobei die Kulturoberfläche Vertiefungen in einer Dichte von mindestens 10 Vertiefungen/cm² oder mehr aufweist.

5. Zellkulturgefäß gemäß einem der Ansprüche 1 bis 4, wobei die Trennwand einen Schlitz und/oder ein Loch aufweist.

6. Zellkulturgefäß gemäß einem der Ansprüche 1 bis 5, wobei die Trennwand kontinuierlich ausgebildet ist.

7. Zellkulturgefäß gemäß einem der Ansprüche 1 bis 6, wobei sich die Trennwand von der Kulturoberfläche entlang einer Richtung senkrecht zu einer Montageoberfläche des Bodens nach oben erstreckt.

8. Zellkulturgefäß gemäß einem der Ansprüche 1 bis 7, wobei die Trennwand aus einer Komponente, abtrennbar von dem Boden und der Umfangswand, aufgebaut ist.

9. Zellkulturgefäß gemäß einem der Ansprüche 1 bis 8, wobei die Kulturoberfläche oberflächenbehandelt ist, um die Haftung von Zellen zu inhibieren.

10. Zellkulturgefäß gemäß einem der Ansprüche 1 bis 9, wobei die Trennwand oberflächenbehandelt ist, um die Haftung von Zellen zu inhibieren.

11. Zellkuklturgefäß gemäß einem der Ansprüche 1 bis 10, wobei die Trennwand mit der Umfangswand verbunden ist.

## Revendications

1. Récipient de culture cellulaire (10) comprenant :
un fond (12) ayant une surface de culture (3) avec une pluralité de puits (2) ;
une paroi périphérique (11) s'étendant vers le haut à partir d'une périphérie du fond (12) ; et
une cloison (15) séparant une région sur la surface de culture entourée par la paroi périphérique (11) en une pluralité de sous-régions,
dans lequel la surface de culture entre les puits adjacents les uns aux autres et entre la paroi périphérique adjacente au puits et le puits est composée d'une surface incurvée continue sans région plate.

2. Récipient de culture cellulaire selon la revendication 1, dans lequel une hauteur de la cloison est dans une plage de 0,5 à 90 % d'une hauteur de la paroi périphérique par référence au fond.

3. Récipient de culture cellulaire selon la revendication 1 ou 2, dans lequel une aire de l'une des sous-régions est dans une plage de 5 à 80 % d'une aire de la région.

4. Récipient de culture cellulaire selon l'une quelconque des revendications 1 à 3, dans lequel la surface de culture a des puits à une densité d'au moins 10 puits/cm² ou plus.

5. Récipient de culture cellulaire selon l'une quelconque des revendications 1 à 4, dans lequel la cloison a une fente et/ou un trou.

6. Récipient de culture cellulaire selon l'une quelconque des revendications 1 à 5, dans lequel la cloison est formée en continu.

7. Récipient de culture cellulaire selon l'une quelconque des revendications 1 à 6, dans lequel la cloison s'étend vers le haut à partir de la surface de culture le long d'une direction perpendiculaire à une surface de montage du fond.

8. Récipient de culture cellulaire selon l'une quelconque des revendications 1 à 7, dans lequel la cloison se compose d'un composant séparable du fond et de la paroi périphérique.

9. Récipient de culture cellulaire selon l'une quelconque des revendications 1 à 8, dans lequel la surface de culture est traitée en surface pour inhiber l'adhésion des cellules.

10. Récipient de culture cellulaire selon l'une quelconque des revendications 1 à 9, dans lequel la cloison est traitée en surface pour inhiber l'adhésion des cellules.

11. Récipient de culture cellulaire selon l'une quelconque des revendications 1 à 10, dans lequel la cloison est reliée à la paroi périphérique.
